# EUROPEAN PATENT APPLICATION

(11) **EP 2 868 781 A1**
(43) Date of publication of application: **06.05.2015**
(21) Application number: 14191657.7
(22) Date of filing: 04.11.2014
(51) Int. Cl.: D01D 5/00, A61M 37/00

(54) **METHOD FOR FABRICATION OF POROUS FIBROUS MICROSTRUCTURE WITH VARIOUS 3-DIMENSIONAL STRUCTURES**

(30) Priority: 04.11.2013 KR 20130133083
(71) Applicant: Industry-Academic Cooperation Foundation Yonsei University, Seoul 120-749 (KR)
(72) Inventor: Il Jung, Hyung, Seoul 122-080 (KR); Li, Cheng Guo, Gyeonggi-do 440-808 (KR)
(74) Representative: Plougmann & Vingtoft A/S

(57) **Abstract**

The present invention relates to a method for fabricating a three-dimensional porous fibrous microstructure, various three-dimensional porous fibrous microstructures fabricated by the method, an apparatus for detecting a biological marker and a drug delivery system comprising the microstructure. The porous fibrous microstructure of the present invention has excellent interconnectivity between pores and micropores and captures and delivers target particles at high efficiency, and thus can be usefully applied to biomedical applications including the detection of a biomarker and drug delivery.

## Description

### BACKGROUND OF THE INVENTION

### FIELD OF THE INVENTION

The present invention relates to methods for fabrication of porous fibrous microstructure using electrospinning and molding method.

### BACKGROUND OF TECHNIQUE

Electrospinning is regarded as the most effective and versatile method for the preparation of ultra-fine polymer fibers based on different polymeric materials. Polymer fibers produced from electrospinning have formed porous structure with excellent pore interconnectivity and the pores are in micrometer to nanometer range. [1] Compared with other one-dimensional nanostructures (e.g. nanotubes, nano-rods, wires), fibers have the open pore structure and high surface area to volume ratio, which is an important property for applications in drug delivery, wound dressing, tissue engineering, sensors and other biomedical purposes. [2-5]

However, the microstructure with desired shape may not be obtained solely by electrospinning. Although the methods for fabricating three-dimensional structure through rapid prototyping and controlling the condition of electrospinning have been suggested, there are obstacles against producing structures of various shapes, especially three-dimensional microstructure (G. Kim, Macromol. Rapid Commun. 29: 15771581 (2008), M. Yousefzadeh, Journal of Engineered Fibers and Fabrics. 7: 17-23 (2012), B.A. Blakeney, Biomaterials 32: 1583-1590 (2011)).

The aim of the present invention is to provide methods for direct fabrication of three-dimensional structured electrospun fiber. The three-dimensional fiber was fabricated by a novel air suction systems combine with collector mold. The air suction system is more effective and convenience method for preparation of various 3-D structured polymer fibers based on different collector mold. Drugs ranging from antibiotics and anticancer agents to proteins, DNA, and RNA can be encapsulated into polymer fibers to apply in drug delivery, wound dressing, tissue engineering and sensors.

Biodegradable polymer microneedles encapsulating drug were designed for controlled release in skin as an alternative to hypodermic injection or implantation of controlled-release systems.[6] Polymeric drug delivery systems have numerous advantages compared to conventional dosage forms, such as improved therapeutic effect, reduced toxicity, convenience, and so on. However, there are still many problems for researchers to solve in the sustained drug delivery using biodegradable polymer microneedles. For example, the microneedles was encapsulated by drug-loaded nano- or microparticles for sustain drug release. However, the efficiency of preparing nano- or microparticles or vesicles is too low and fabrication methods have often been time consuming and expensive due to reliance on multi-step.[7] Moreover, arrays of microneedles also were fabricated out of polylactic acid, polyglycolic acid, and their co-polymers using a mold-based technique to encapsulate high concentration model drugs, however, it need to melt the polymer under high temperature which was not suitable for protein drug.[8-9]

The present inventors have combined electrospun fiber with dissolving microneedle to fabricate the novel fibrous microstructure for sustained drug delivery for the first time. Microstructure for sustained release can be fabricated by direct capsulation of drugs into electrospun fibers having low rate of degradation and dissolution. In addition, soluble and biodegradable polymer with high mechanical strength may be combined into the porous structure in cases sufficient mechanical strength is needed, e.g. skin penetration. Through these processes, various type of 3D electrospun fibrous microstructure with sufficient mechanical strength may be produced without morphological change. Along with degradation of the fibers, the drug which was encapsulated inside fibers was sustained released. Therefore, such 3D structured polymer fibers and biodegradable microstructure with ultrafine fiber will be promising in the future biomedical applications.

Throughout this application, various publications and patents are referred and citations are provided in parentheses. The disclosures of these publications and patents in their entities are hereby incorporated by references into this application in order to fully describe this invention and the state of the art to which this invention pertains.

### SUMMARY OF THE INVENTION

The present inventors have made intensive studies to develop an efficient process for fabricating various forms of three-dimensional porous fibrous microstructures having a high ratio of volume versus surface area and excellent pore interconnectivity. As results, the present inventors have discovered that, in the case where a fibrous polymer obtained through electro-spinning from a solution in which hydrophilic and hydrophobic polymers are variously mixed is collected in a three-dimensional mold, and then dried and separated, the micropores formed between fibers are connected to each other in an opened state while having a large internal surface area, thereby a fibrous microstructure which has a three-dimensional structure having the same appearance as the mold and having internal characteristics useful in biomedical applications may be obtained.

Accordingly, it is an object of this invention to provide a method for fabricating a three-dimensional porous fibrous microstructure

It is another object of this invention to provide a three-dimensional porous fibrous microstructure fabricated by the method according to this invention.

It is still another object of this invention to provide an apparatus for detecting a biological marker comprising the three-dimensional porous fibrous microstructure according to this invention.

It is still another object of this invention to provide a drug delivery system comprising the three-dimensional porous fibrous microstructure according to this invention.

Other objects and advantages of the present invention will become apparent from the following detailed description together with the appended claims and drawings.

### BRIEF DESCRIPTION OF THE DRAWINGS

Fig. 1 represents a method to fabricate fibrous microstructure that encapsulate drug for sustained controlled release. (i) The single polymer fiber; (ii) the hybrid fiber in which hydrophilic and hydrophobic polymers are entangled; or (iii) core-shell fiber with hydrophilic polymer shell and hydrophobic polymer core.
Fig. 2 shows SEM (Fig. 2a) and TEM (Fig. 2b) image of a coaxial fiber of PVP/PLGA. The arrows indicate the core and shell parts within the fiber. The arrows in Fig. 2b indicate the core and shell parts within the fiber.
Fig. 3 shows laser scanning confocal microscopy images of blend and coaxial PVP/PLGA electrospun fiber.
Fig. 4 represents cylinder shape of collector mold (Fig. 4a); optical microscope image of cylinder PLGA fibers (Fig. 4b); and SEM image of PLGA fibers (Fig. 4c), respectively.
Fig. 5 represents cone shape of collector mold (Fig. 5a); optical microscope image of cone PLGA fibers (Fig. 5b); and SEM image of PLGA fibers (Fig. 5c), respectively.
Fig. 6 represents hemisphere shape of collector mold (Fig. 6a); optical microscope image of hemisphere PLGA fibers (Fig. 6a); and SEM image of PLGA fibers (Fig. 6c), respectively.

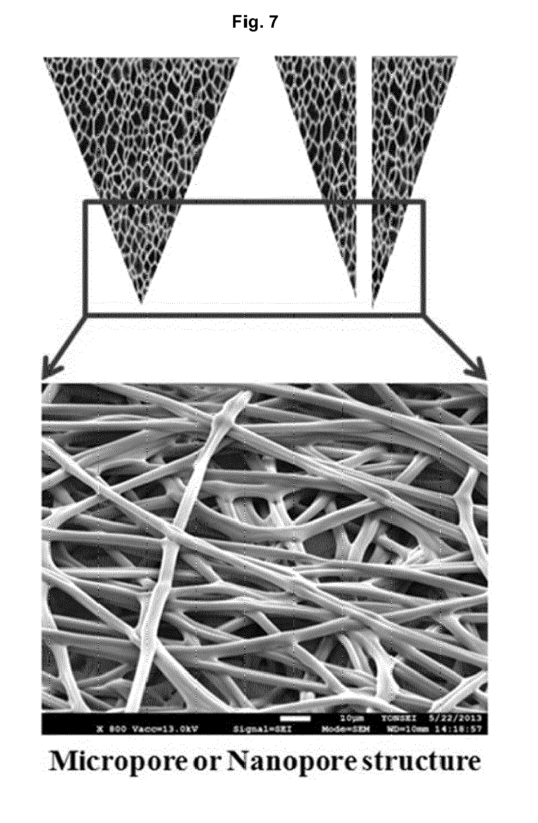

Fig. 7 represents the porous microstructure for detection and drug delivery.

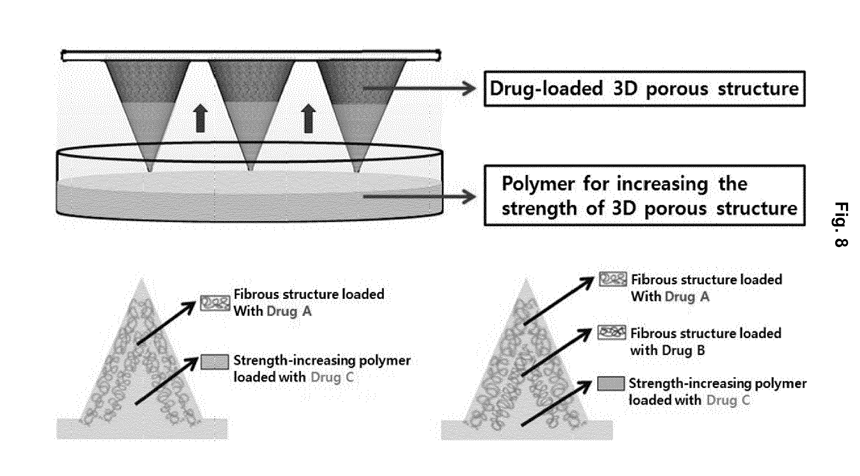

Fig. 8 represents a scheme for process of combining other polymer into fibrous microstructure to increase mechanical strength. Additional polymer solution for mechanical strength may be loaded. Through loading further drugs into the polymer solution, finally the second and the third drugs may be encapsulated in the 3D fibrous microstructure.

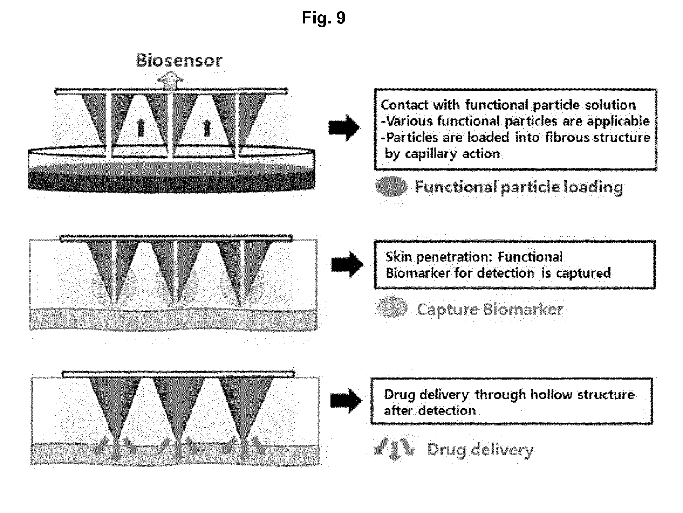

Fig. 9 represents a scheme for process of loading functional particle inside 3D fibrous microstructure.

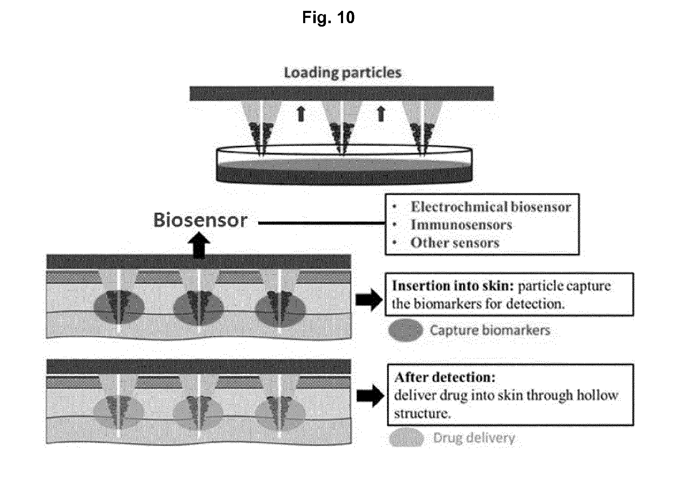

Fig. 10 represents a process of loading functional particle inside hollow fibrous microstructure for detection and drug delivery.
Fig. 11a shows a basic process to fabricate fibrous microstructure that encapsulate drug for sustained release. Figs. 11b-11e show exemplary embodiments of the present invention adopting the fixing thin plate to allow polymer fiber to be inserted into the molds evenly during centrifugation.
Fig. 12 represents a scheme for process to fabricate fibrous microstructure that encapsulate drug for sustained controlled release by applying downward pressure.
Fig. 13 represents a scheme for process to obtain 3D fibrous microstructure by etching method in which fibrous gel is dried and solidified, and then separated from the mold.
Fig. 14 shows optical microscope image (Fig. 14a), SEM image (Fig. 14b) and confocal microscope image (Fig. 14c) of fibrous microstructure, respectively.
Fig. 15 shows optical microscope image (Fig. 15a), SEM image (Fig. 15b) and confocal microscope image (Fig. 15c) of porous fibrous microneedle, respectively.

### DETAILED DESCRIPTION OF THE INVENTION

In one aspect of this invention, there is provided a method for fabricating a three-dimensional porous fibrous microstructure, comprising:
(a) injecting a polymer solution into an injecting member including a syringe pump and a spinneret;
(b) spinning the polymer solution, which is injected into the injecting member, through the spinneret using the syringe pump together with the application of voltage, thereby obtaining a polymer fiber;
(c) collecting the polymer fiber into a mold; and
(d) drying the polymer fiber, and then separating the dried polymer fiber from the mold, thereby obtaining a three-dimensional porous fibrous microstructure.

As results, the present inventors have discovered that, in the case where a fibrous polymer obtained through electro-spinning from a solution in which hydrophilic and hydrophobic polymers are variously mixed is collected in a three-dimensional mold, and then dried and separated, the micropores formed between fibers are connected to each other in an opened state while having a large internal surface area, thereby a fibrous microstructure which has a three-dimensional structure having the same appearance as the mold and having internal characteristics useful in biomedical applications may be obtained.

According to the present invention, the fibrous microstructure fabricated by the method of the present invention has excellent drug delivery while collecting and detecting a disease marker at high sensitivity in the skin, the body fluid, and the like.

### Step (a): Injecting polymer solution into injecting member

As used herein, the term "injecting member" refers to a tool which contains a polymer solution of the present invention and spins the polymer solution through a spinneret with a predetermined diameter, using an injection pressure.

As used herein, the term "polymer solution" refers to a raw material of the porous fibrous microstructure fabricated by the method of the present invention, and includes various hydrophilic or hydrophobic polymer solutions that can form a fibrous microstructure through electro-spinning. According to the present invention, the polymer solution of the present invention can be appropriately selected by various combinations in order to control the density, strength, and distribution of micropores of the microstructure to be obtained.

The polymer solution used herein may be hydrophilic or hydrophobic. The hydrophilic polymer solution includes, for example, sodium carboxymethyl cellulose (CMC), polyvinyl pyrrolidone (PVP), hyaluronic acid (HA), polyvinyl alcohol (PVA), and hydroxypropylmethyl cellulose (HPMC), but any hydrophilic polymer that can form a fibrous structure through electrospinning and can be conventionally used as a hydrophilic polymer in the art may be used without limitation thereto.

The hydrophobic solution includes, for example, poly(lactic-co-glycolic acid) (PVA), but any hydrophobic polymer that can form a fibrous structure through electrospinning and can be conventionally used as a hydrophobic polymer in the art may be used without limitation thereto.

The viscosity of the polymer solution may be variously changed depending on the kinds, concentrations, or temperatures of materials contained in the solution, the addition of a viscosity modifying agent, and the like, and may be appropriately controlled for the purpose of the present invention.

For example, a viscosity modifying agent conventionally used in the art, such as hyaluronic acid or a salt thereof, polyvinyl pyrrolidone, cellulosic polymers, dextran, gelatin, glycerin, polyethylene glycol, polysorbate, propylene glycol, povidone, carbomer, ghatti gum, guar gum, glucomannan, glucosamine, dammer resin, rennet casein, locust bean gum, microfibrillated cellulose, psyllium seed gum, xanthan gum, arabino galactan, Arabic gum, alginates, gelatin, gellan gum, carrageenan, karaya gum, curdlan, chitosan, chitin, tara gum, tamarind gum, tragacanth gum, furcelleran, pectin, or pullulan, may be added to the polymer solution, which is a main component of the microstructure, thereby appropriately controlling the viscosity of the polymer solution for the purpose of the present invention.

### Step (b): Electrospinning

According to the present invention, the polymer solution injected into the injecting member is spun through a spinneret under a predetermined voltage by using a syringe pump. The solution-state polymer becomes a fibrous polymer through the electrospinning, and then collected in a mold. A high voltage of a predetermined range for implementing electrospinning is applied between the spinneret and the mold for collection, and the specific range of voltage is 1-30 kV, more specifically, 5-20 kV, and the most specifically, 9-15 kV.

### Step (c): Collecting polymer fiber into mold

The polymer fiber obtained through electrospinning may be collected into the mold by various methods. Specifically, a downward pressure is applied to the gel-state coated on a mold substrate, thereby pushing the fibrous polymer into the mold. On the contrary, the mold may be filled with the polymer fiber by positioning the mold above the fibrous polymer coated on the substrate and applying a downward pressure to the mold. Alternately, the mold may be filled with the fibrous polymer by applying centrifugal force to the mold coated with the fibrous polymer. Last, the mold may be uniformly filled with the fibrous polymer by applying a negative pressure. The negative pressure may be applied in various manners known in the art, and for example, the negative pressure may be applied by sucking the fibrous polymer in the mold through an air suction system which includes a vacuum pump connected with a space inside the mold. The electrospinning and the collecting of the fibrous polymer into the mold may be sequentially carried out or may be simultaneously carried out. Therefore, step (b) and step (c) herein may be sequentially performed or may be simultaneously performed.

### Step (d): Separating polymer fiber from mold

According to the method of the present invention, the fiber with which the mold is filled is solidified through drying, and then separated from the mold, thereby obtaining a porous fibrous microstructure having the same structure as the mold. This procedure is a last stage of a molding method conventionally used in the art, and may be performed under the appropriate reagent and temperature conditions in which a casting and a mold are capable of being separated from each other.

The mold used herein may be variously selected depending on the shape of a desired three-dimensional porous fibrous microstructure, and may have, for example, a cylinder shape, a cone shape or a hemisphere shape. Therefore, by using the present invention, porous fibrous microstructures having various three-dimensional appearances, which are appropriate for the purpose of the present invention, can be obtained. However, in the prior art, it was impossible to freely control the appearance of a structure using fibers obtained by electrospinning.

According to a specific embodiment of the present invention, the polymer solution of the present invention corresponds to (i) a single polymer solution injected into a single injecting member; (ii) two or more polymer solutions which are respectively injected into two or more injecting members and individually spun through spinnerets of the respective injecting members; or (iii) two or more polymer solutions which are respectively injected into two or more injecting members and spun through a coaxial spinneret in which a spinneret of one injecting member is inserted into a spinneret of the other injecting member.
(i) In the case of the single polymer solution injected into the single injecting member, a single component of fiber is formed through electrospinning and the fiber is collected into the mold, thereby obtaining a single component of porous fibrous microstructure.
(ii) In the case of the two or more polymer solutions which are respectively injected into two or more injecting members and individually spun through spinnerets of the respective injecting members, respective fibers of different components are collected in the mold while being mixed with each other, thereby obtaining a complex component of porous fibrous microstructure. For example, in the case where a first injecting member into which a hydrophilic polymer solution is injected and a second injecting member into which a hydrophobic polymer solution is injected are electrospun, the respective hydrophilic and hydrophobic fibers are collected into the same mold while being entangled with each other to form a hybrid fiber.
(iii) In the case of the coaxial spinneret, with respect to the two or more injecting members into which two or more polymer solutions are respectively injected, one spinneret nozzle is inserted into the other spinneret nozzle, thereby obtaining a core-shell fiber as a result of electrospinning.

According to a specific embodiment of the present invention, the polymer solution of the present invention corresponds to (i) a single polymer solution injected into a single injecting member; (ii) a hydrophilic polymer solution and a hydrophobic polymer solution which are respectively injected into two or more injecting members and individually spun through spinnerets of the respective injecting members; or (iii) a hydrophilic polymer solution and a hydrophobic polymer solution which are respectively injected into two or more injecting members and spun through a coaxial spinneret in which a spinneret of one injecting member is inserted into a spinneret of the other injecting member.

According to a specific embodiment of the present invention, step (c) of the present invention is performed by contacting the mold with a fiber sheet loading the polymer fiber while applying a downward pressure to the mold.

In another aspect of this invention, there is provided a method for fabricating a three-dimensional porous fibrous microstructure, comprising:
(a) injecting a hydrophilic polymer solution and a hydrophobic polymer solution into two injecting members including syringe pumps and spinnerets;
(b) individually spinning the hydrophilic polymer solution and the hydrophobic polymer solution, which are injected into the respective injecting members, through the spinnerets using the syringe pumps together with the application of voltage, thereby obtaining a hydrophilic polymer fiber and a hydrophobic polymer fiber;
(c) collecting the hydrophilic polymer solution and the hydrophobic polymer into a mold;
(d) drying the polymer fibers, and then separating the dried polymer fibers from the mold, thereby obtaining a hybrid fibrous microstructure; and
(e) etching the hybrid fibrous structure with a water-soluble solvent, thereby obtaining a three-dimensional porous microstructure.

Since the injecting members, the polymer solutions, the electrospinning procedure, the collecting procedure into the mold, and the separating procedure from the mold have been previously described, the descriptions thereof will be omitted to avoid excessive overlapping. Also in the present embodiment, the electrospinning and the collecting of the polymer fiber into the mold may be sequentially carried out or may be simultaneously carried out. Therefore, step (b) and step (c) herein may be sequentially performed or may be simultaneously performed.

According to the present invention, the microstructure obtained from the hydrophilic and hydrophobic hybrid fiber is subjected to a further etching process to leave only a hydrophobic fiber portion, thereby obtaining a porous fibrous microstructure having an increased space for loading drugs or collecting a biological material (e.g., a disease marker in the body fluid). As the water-soluble solvent used in the etching process, any solvent that can dissolve to corrode only a hydrophilic fiber may be used, and for example, water, or anhydrous or hydrous lower alcohol having 1-4 carbon atoms may be used.

In still another aspect of this invention, there is provided a method for fabricating a three-dimensional porous fibrous microstructure, comprising:
(a) injecting a polymer solution into an injecting member including a syringe pump and a spinneret;
(b) spinning the polymer solution, which is injected into the injecting member, through the spinneret using the syringe pump together with the application of voltage, thereby obtaining a polymer fiber;
(c) collecting the polymer fiber into a mold;
(d) drying the polymer fiber, and then separating the dried polymer fiber from the mold, thereby obtaining a three-dimensional porous fibrous microstructure; and
(e) contacting the obtained three-dimensional porous fibrous microstructure with a high-strength polymer solution to allow the high-strength polymer solution to be loaded on the three-dimensional porous fibrous microstructure, thereby obtaining a three-dimensional porous fibrous microstructure having an enhanced strength.

Since steps (a)-(d) of the present invention overlap those in the above method, the descriptions thereof will be omitted to avoid excessive overlapping.

As used herein, the term "high-strength polymer solution" refers to a solution which contains a polymer which is loaded on the microstructure of the present invention to constitute a part of the structure, thereby giving an enhanced strength.

The present invention can be useful in fabricating a sustained release type microstructure which has a more enhanced strength than the soluble type microstructure having a sustained release property in the prior art. That is, in the prior art, a soluble type microneedle was fabricated by mixing a drug to be delivered with a polymer having soluble/biodegradable characteristics in order to fabricate a sustained release type microneedle, but the strength of the microneedle was not sufficient and thus the microneedle could not penetrate the skin. In addition, in order to overcome the disadvantage, the soluble type microneedles were fabricated by forming micro/nanoparticles of a polymer such as PLGA having a sustained release property, and then mixing the micro/nanoparticles with a polymer having a sufficient strength. However, the procedure was difficult, the yield was low, and a desired strength was not obtained.

However, the polymer solution giving strength is loaded inside the fibrous structure by contacting the polymer solution giving strength with the three-dimensional porous fibrous microstructure of the present invention, thereby finally obtaining a three-dimensional porous fibrous microstructure having a significantly enhanced strength when compared with the strength of the soluble type microneedle of the prior art. The high-strength polymer used herein includes, for example, hyaluronic acid or a salt thereof, polyvinyl pyrrolidone, cellulose polymer, dextran, gelatin, glycerin, polyethylene glycol, polysorbate, propylene glycol, povidone, carbomer, ghatti gum, guar gum, glucomannan, glucosamine, dammer resin, rennet casein, locust bean gum, microfibrillated cellulose, psyllium seed gum, xanthan gum, arabino galactan, Arabic gum, alginates, gelatin, gellan gum, carrageenan, karaya gum, curdlan, chitosan, chitin, tara gum, tamarind gum, tragacanth gum, furcelleran, pectin, and pullulan, but is not limited thereto. More specifically, the viscous material included in the high-strength polymer used herein is a cellulose polymer, more specifically, hydroxypropyl methylcellulose, hydroxyalkyl cellulose (specifically, hydroxyethyl cellulose or hydroxypropyl cellulose), ethyl hydroxyethyl cellulose, alkyl cellulose, or carboxymethyl cellulose, still more specifically, hydroxypropyl methyl cellulose or carboxymethyl cellulose, and most specifically, carboxymethyl cellulose. The polymer solution in step (a) or the high-hardness polymer solution in step (e) of the present invention may include a drug to be delivered, or both the polymer solution in step (a) and the high-hardness polymer solution in step (e) may include a drug to be delivered. The latter may be the same drug or different drugs (a first drug and a second drug) depending on the purpose.

In still another aspect of this invention, there is provided a three-dimensional porous fibrous microstructure fabricated by the method of the present invention.

In still another aspect of this invention, there is provided an apparatus for detecting a biological marker, the apparatus including the three-dimensional porous fibrous microstructure of the present invention.

Since the three-dimensional porous fibrous microstructure used herein has been described, the descriptions thereof will be omitted to avoid excessive overlapping.

The three-dimensional fibrous microstructure of the present invention has high porosity and excellent pore interconnectivity, and thus the fluid can be sucked through a capillary phenomenon due to micro-scale void spaces. Due to the reason, since the present invention can load particles capable of detecting a biological marker, the biological marker can be detected at high efficiency by merely contacting the microstructure of the present invention with a sample to be detected or allowing the sample to be detected to invade the microstructure of the present invention.

According to a specific embodiment of the present invention, with respect to the apparatus of the present invention, a sensor for the biological marker is connected to the porous fibrous microstructure.

According to the present invention, the material to be detected in a biological sample can be detected by using the disease marker detected in the skin and the body fluid. Further, a three-dimensional microstructure for electric signal transmission is constructed, thereby eventually enabling a high-sensitivity and real-time diagnosis. As used herein, the term "sensor for the biological marker" refers to any biosensor which is connected to the porous fibrous microstructure of the present invention to detect and analyze the biological marker in real time or sequentially, and includes, for example, an electrochemical biosensor and an immune sensor such as an antibody.

In still another aspect of this invention, there is provided a drug delivery system including the three-dimensional porous fibrous microstructure of the present invention.

Since the three-dimensional porous fibrous microstructure used herein has been described, the descriptions thereof will be omitted to avoid excessive overlapping.

The three-dimensional porous fibrous microstructure of the present invention has high porosity and excellent pore interconnectivity, and thus can load particles of a drug at high efficiency by merely a simple contact with the drug. Thus, the microstructure capturing a drug is allowed to invade a lesion site, such as the skin, thereby easily delivering the drug.

The features and advantages of the present invention will be summarized as follows:
(a) The present invention provides a method for fabricating a three-dimensional porous fibrous microstructure, various three-dimensional porous fibrous microstructures fabricated by the method, an apparatus for detecting a biological marker and a drug delivery system comprising the microstructure.
(b) The porous fibrous microstructure of the present invention has excellent interconnectivity between pores and micropores and captures and delivers target particles at high efficiency, and thus can be usefully applied to biomedical applications including the detection of a biomarker and drug delivery.
(c) The three-dimensional porous fibrous microstructure of the present invention is used in detecting the immobilized disease marker as well as constructing a fibrous microstructure for electric signal transmission, thereby eventually enabling simultaneous multi-marker detection at high sensitivity.
(d) The porous fibrous microstructure of the present invention is fabricated in a form of an integration type sensor as an electrode capable of transmitting an electric signal, thereby detecting a disease marker in the body fluid in real time.

The present invention will now be described in further detail by examples. It would be obvious to those skilled in the art that these examples are intended to be more concretely illustrative and the scope of the present invention as set forth in the appended claims is not limited to or by the examples.

### EXAMPLES

### MATERIALS AND METHODS

### Air Suction system for three-dimensional electrospun-fiber

First, a suspension of drug is mixed into a polymer solution. In the electrospinning process, evaporation of the solvent results into fiber laden with homogeneously distributed drug. (i) The single polymer fiber or (ii) The hydrophilic and hydrophobic polymer from two individual ports entangle by crossing over each other forming hybrid fiber or (iii) core-shell fiber with hydrophilic polymer shell and hydrophobic polymer core are collected and filled into the mold under the air suction systems. Finally, drying and separation from the mold results into the three-dimensional electrospun fiber microstructure.

### Fabrication of three-dimensional PLGA fiber microstructure

For the preparation of the composite solutions used in electro-spinning process, the poly (lactic-co-glycolic acid) PLGA (85:15, MW:50000~70000) dissolved in hexafluoro-2-isopropanol (HFIP), and the PLGA solution with concentration of 15% w/v was obtained under gentle stirring for 1 h at room temperature. In this study, the electrospinning apparatus consisted of an infusion pump, high voltage power supply and a grounded target. The composite solution was fed into a 5 mL plastic syringe fitted with a stainless-steel blunt needle of 0.33 mm in diameter and an injection rate of 3 mL/h using an infusion pump. A high voltage of 9 kV was applied to the composite solution. Randomly-oriented PLGA fibers were collected on a collector which was kept at a distance of 12 cm from the needle tip.

### Fabrication of BSA loaded PLGA/PVP (core/shell) three-dimensional fiber microstructure with by coaxial electrospinning

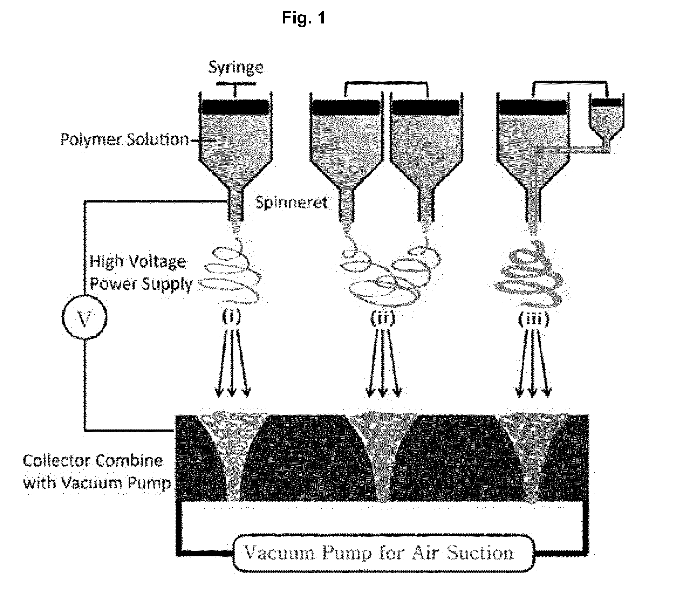

For the preparation of the composite solutions used in electro-spinning process, the PLGA dissolved in hexafluoro-2-isopropanol (HFIP) with concentration of 15% w/v and PVP dissolved in ethanol with concentration of 9% w/v. Both of them were obtained under gentle stirring for 1 h at room temperature. The PVP (hydrophilic polymer) was used as a shell material loading with Cy-3 labeled BSA for constructing a core-shell fibrous membrane. PLGA loading with Cy-5 labeled BSA formed the core section of the core-shell fibers. In this experiment, the core-shell fibers were prepared using the co-axial electrospinning. The experimental setup for coaxial electrospinning is shown in Figure 1 (iii). Both the shell solution and core solution were fed independently with a programmable syringe pump. The feed rates are both set at 4 ml/hour and applied voltage was 15 kV. Fibers were collected on the mold which connected with vacuum pump for suction fiber inside the mold.

### Characterization of three-dimensional electrospun fiber microstructure

The morphology of the electrospun fibers was studied with an optical microscope and emission scanning electron microscope (Model JEOL-7001). For SEM, each fiber sample was coated with gold using a sputtering machine for 200s prior to observation under the SEM at an accelerating voltage of 15 kV.

The corer/shell fiber structure in which BSA is incorporated was examined using a JEOL 2010 transmission electron microscope, operated at 60 kV. The samples for TEM were prepared by direct deposition of the electrospun fibers onto copper grids. To visualize the presence and distribution of the proteins in the electrospun fibers, samples for confocal microscopy were prepared using Cy3-BSA and Cy5-BSA (50 µg/ml) to stain the polymer PVP and PLGA, respectively. A thin layer of electrospun fibers was collected on a glass slide and then observed by Laser scanning confocal microscopy (LSCM). The excitation wavelengths for Cy3 and Cy5 were 550 and 649 nm, respectively.

### RESULTS

### Morphology of the electrospun fibers

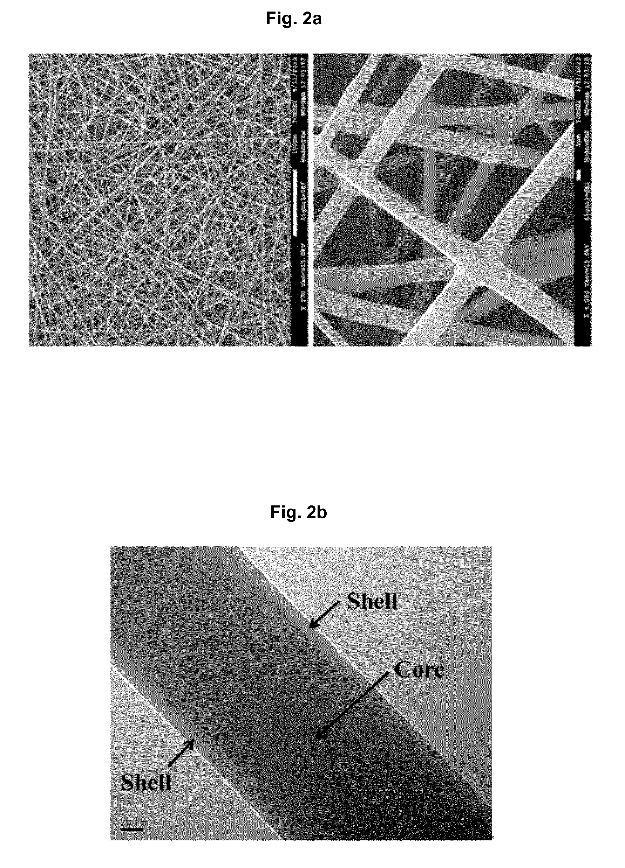

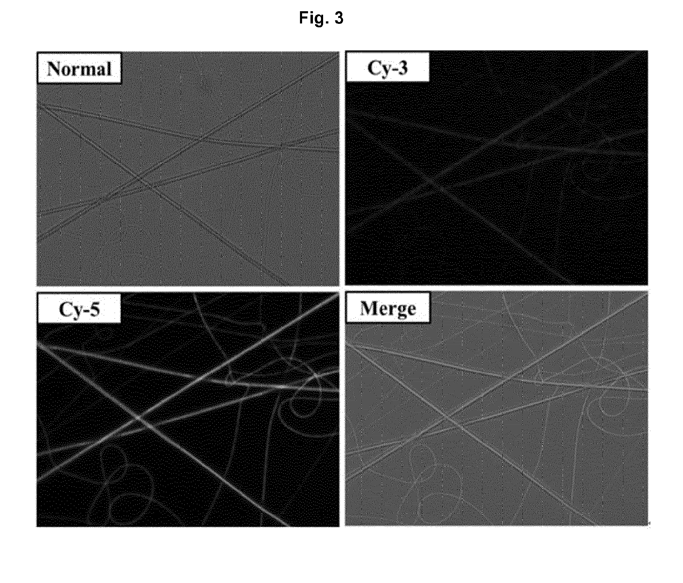

(1) SEM images showed an irregular fiber morphology, whereas the coaxially electrospun PVP/PLGA fibers revealed a relatively uniform fiber morphology (Figure 2a). TEM demonstrated that the coaxially electrospun fibers exhibited an obvious core-shell structure (Figure 2b), indicating the differences in electron density between the inner core and outer shell of the fibers.
(2) LSCM was used to visualize the protein distribution within the electrospun fibers prepared by the two techniques. The green stain can be attributed to Cyanine dyes Cy3 label linked to BSA in the shell solution (fluorescent in the green region), present in the polymer shell solution, whereas the red stain was from the Cy5 label linked to BSA in the core solution (fluorescent in the red region). The coaxially electrospun fibers PVP/PLGA exhibited a relatively homogeneous protein distribution (Figure 3).
(3) Three-dimensional electrospun fiber microstructure

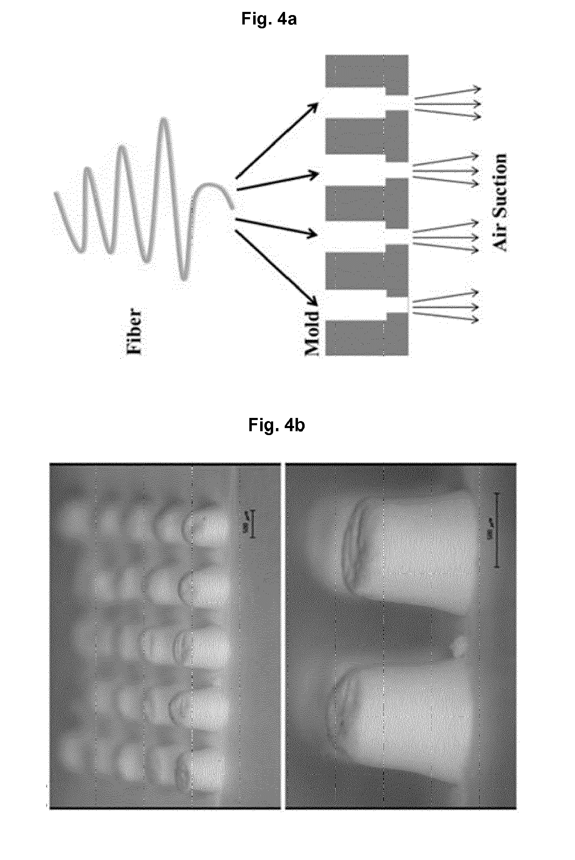

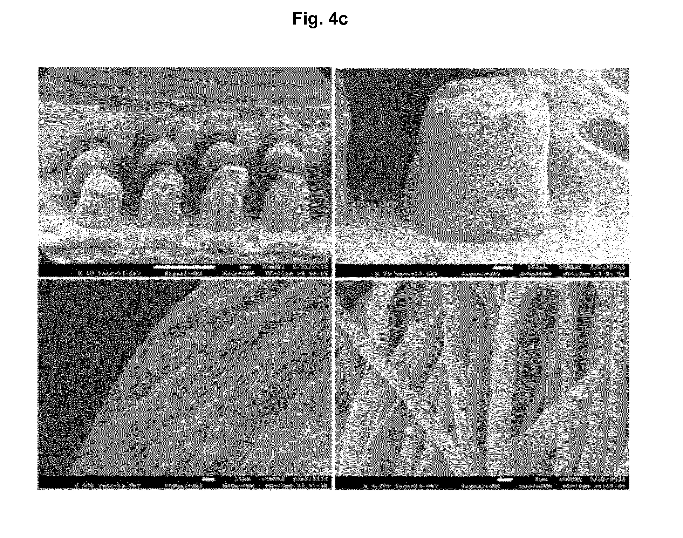

Cylinder shape PLGA 3D fiber structure: In the electrospinning process, formed PLGA fibers were collected and filled into the cylinder shape mold under the air suction systems (Figure 4a). Finally, drying and separation from the mold results into the cylinder three-dimensional electrospun fiber microstructure. Figures 4 b and 4c show the optical and SEM image of the PLGA 3D fiber structure with 1000 µm height and 800 µm base diameters.

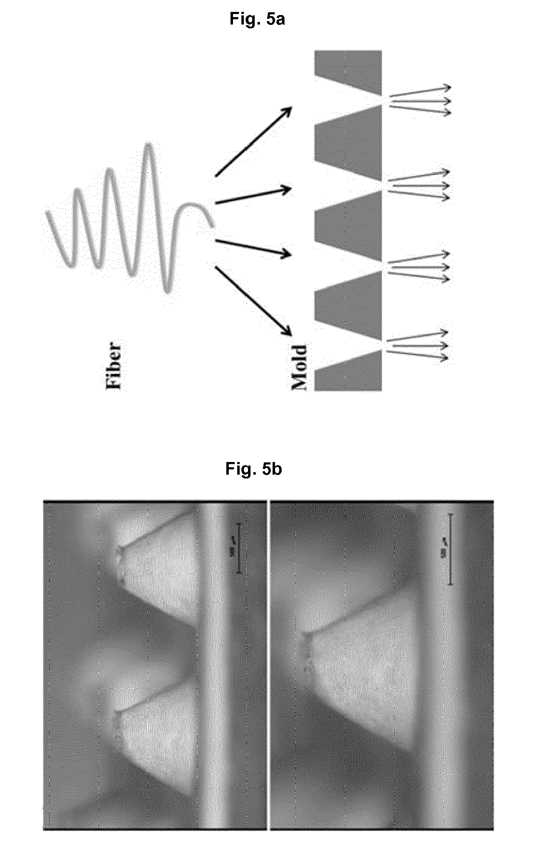

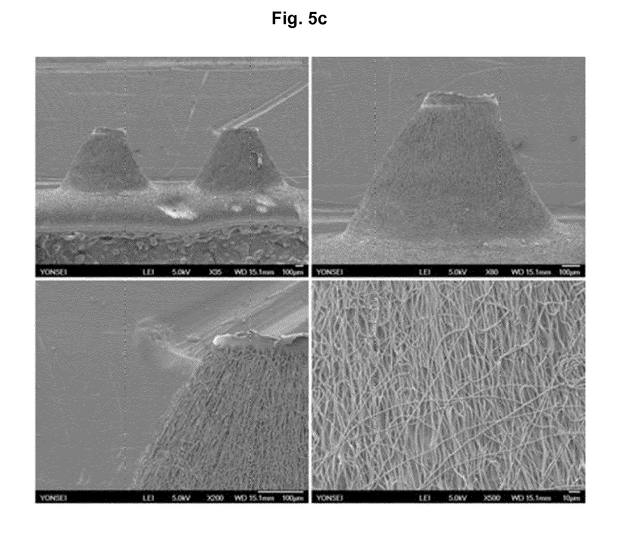

Cone shape PLGA 3D fiber structure: In the electrospinning process, formed PLGA fibers were collected and filled into the cone shape mold under the air suction systems (Figure 5a). Finally, drying and separation from the mold results into the cone three-dimensional electrospun fiber microstructure. Figures 5b and 5c show the optical and SEM image of the PLGA 3D fiber structure with 800 µm height and 800 µm base diameters.

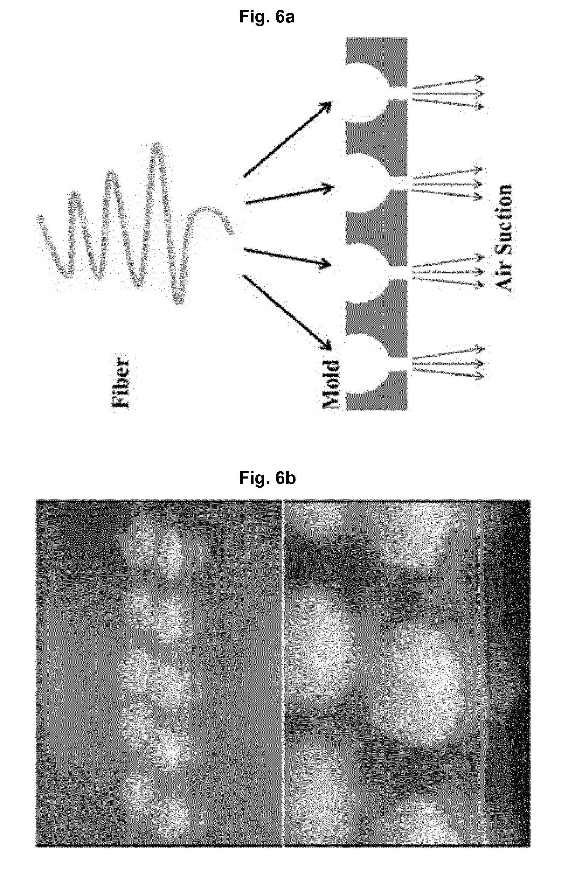

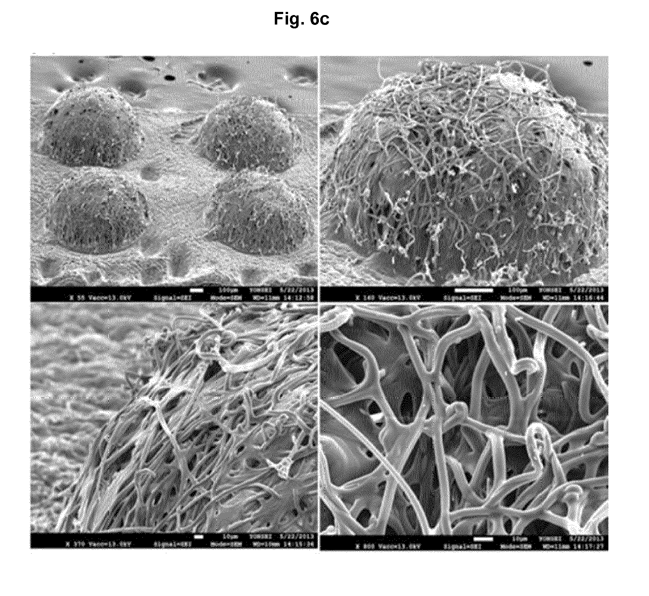

Hemispheres shape PVP/ PLGA 3D fiber structure: In the electrospinning process, formed PVP/ PLGA fibers were collected and filled into the hemispheres shape mold under the air suction systems (Figure 6a). Finally, drying and separation from the mold results into the cone three-dimensional electrospun fiber microstructure. Figures 6b and 6c show the optical and SEM image of the PLGA 3D fiber structure with 500 µm height and 500 µm base diameters.

### Porous three-dimensional electrospun fiber microstructure for electrode-sensor

Conductive polymer such as polyaniline was used to fabricated electrospun fibers. Using these conductive polymers in the present air suction systems, the present inventors fabricated the porous three-dimensional fiber microstructure for electrode sensor. In addition, surface modification of porous fibrous structure also can be used for electrode sensor, such as electroplating or coating conductive materials. This porous structure can increase the contact space for binding antibody, molecule and functional particle for application in detection electrode sensor. As the polymer fibers was produced from electrospinning have formed porous structure with excellent pore interconnectivity and the pores size are in micrometer to nanometer range, it can be easy to convert the bio-information into electronic information under the electronic change with the interaction between biological molecules.

Due to the micro-scale void spaces in the microneedle, it will be able to contact fluid from random skin sites with capillary action. The particle with biomarker can be stored in the pores of micropass, particle interface is separated making a wide application area and possibility of loading any form of particle biomarkers.

### Fibrous dissolving microneedle for sustained drug delivery

### Centrifugation molding method

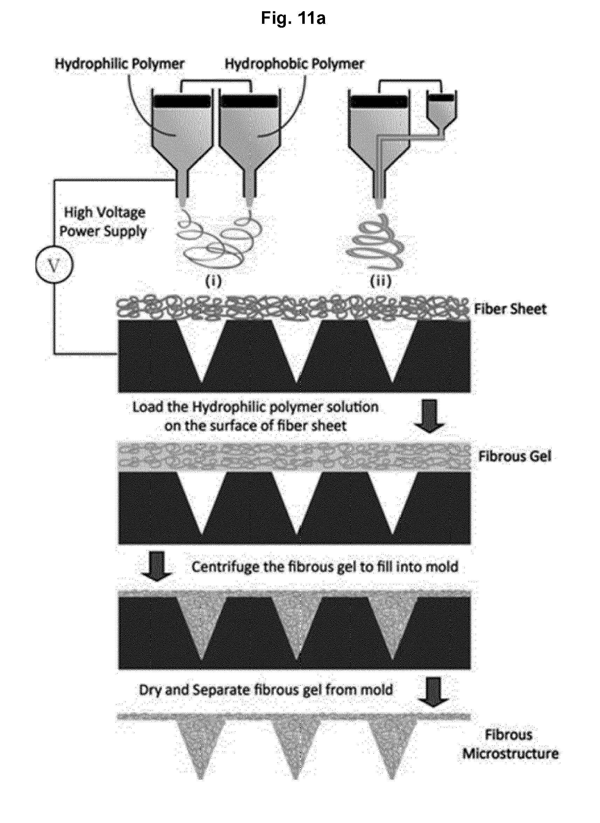

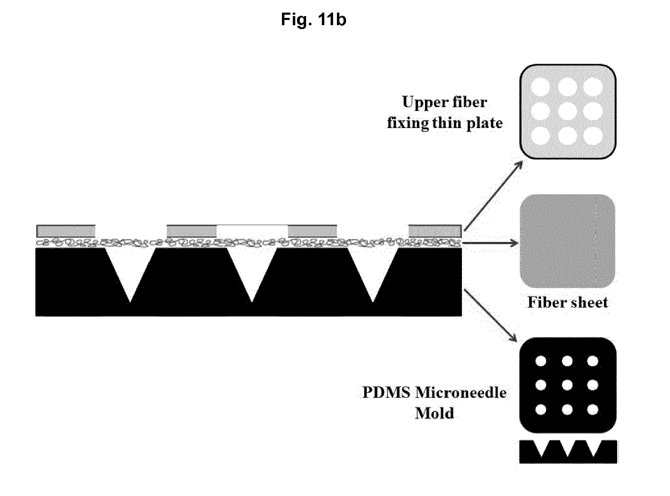

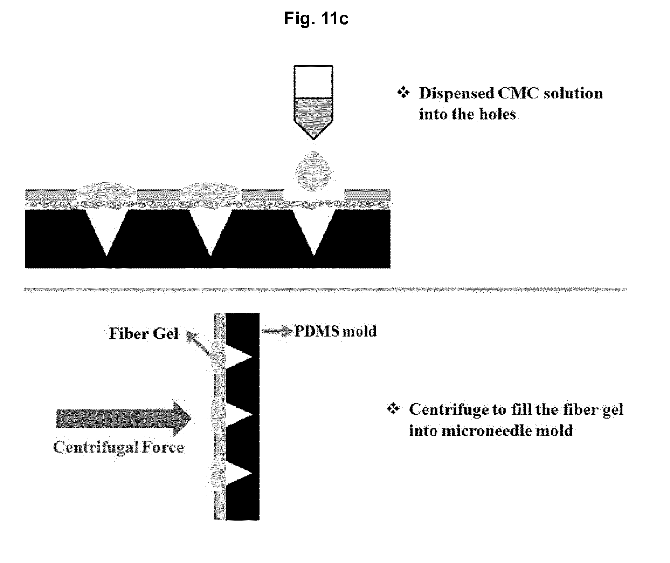

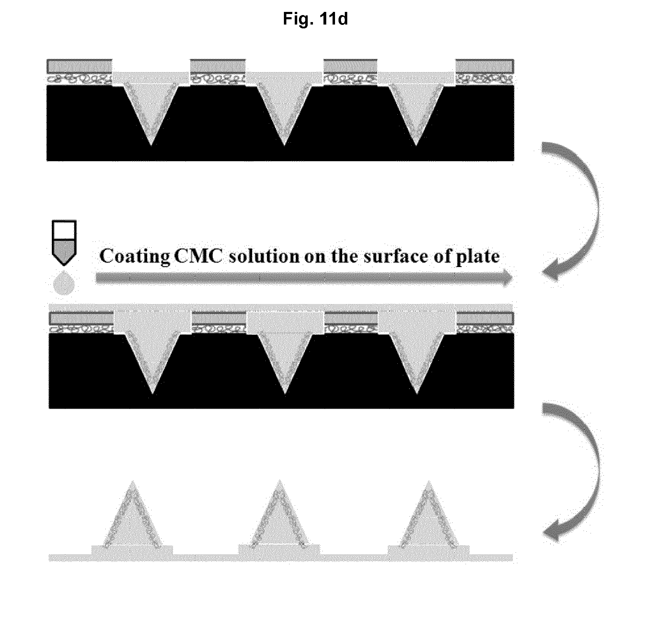

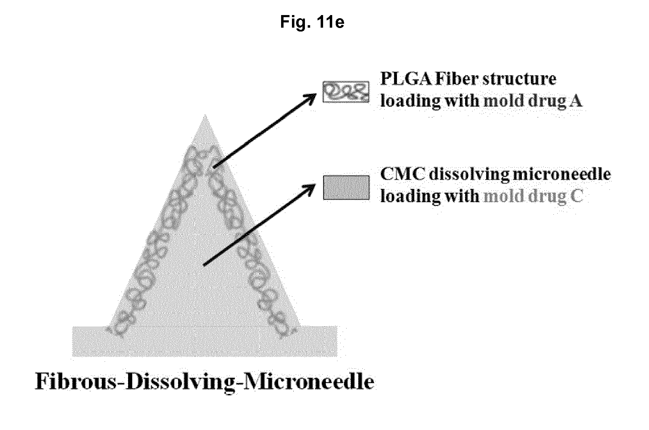

First, a suspension of drug is mixed into a hydrophobic polymer solution. In the electrospinning process, evaporation of the solvent results into fiber laden with homogeneously distributed drug. (i) The hydrophilic and hydrophobic polymer from two individual ports entangle by crossing over each other forming hybrid fiber sheet or (ii) core-shell fiber sheet with hydrophilic polymer shell and hydrophobic polymer core are collected on the surface of mold. Second, the biodegradable polymer solution which was used for dissolving microneedle (such as CMC, HA, PVP and so on) loaded on the fiber sheet to form fibrous gel. Finally, the fibrous gel is then centrifuged into the mold under high centrifugal force. Drying and solidification followed by separation from the mold results into the fibrous microstructure (Figure 11).

### Cast molding method

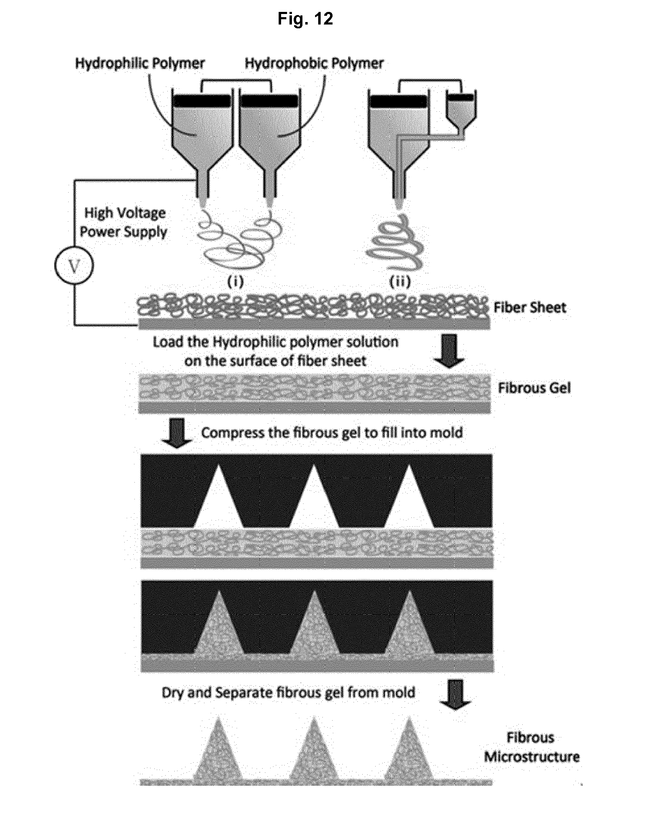

First, a suspension of drug is mixed into a hydrophobic polymer solution. In the electrospinning process, evaporation of the solvent results into fiber laden with homogeneously distributed drug. (i) The hydrophilic and hydrophobic polymer from two individual ports entangle by crossing over each other forming hybrid fiber sheet or (ii) core-shell fiber sheet with hydrophilic polymer shell and hydrophobic polymer core are collected on the surface of plate. Second, the biodegradable polymer solution which was used for dissolving microneedle (such as CMC, HA, PVP and so on) loaded on the fiber sheet to form fibrous gel. Finally, cast mold is pressed to fill fibrous gel into the mold under high compression force. Drying and solidification followed by separation from the mold results into the fibrous microstructure (Figure 12).

### Etching method for three-dimensional electrospun-fiber

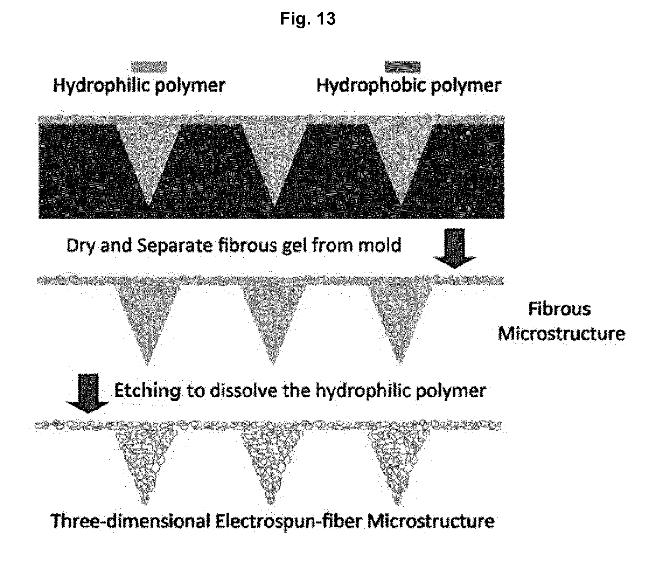

The eaching method was used to dissolve the hydrophilic polymers inside three-dimensional fibrous microstructure such as sodium carboxymethyl cellulose (CMC), polyvinyl pyrrolidone (PVP), hyaluronic acid (HA), polyvinyl alcohol (PVA), hydroxypropylmethyl cellulose (HPMC) and so on. Thus, the hydrophobic fibers inside are left due to the prolonged biodegradability result in the three-dimensional fiber microstructure (Figure 13).

### EXPERIMENT

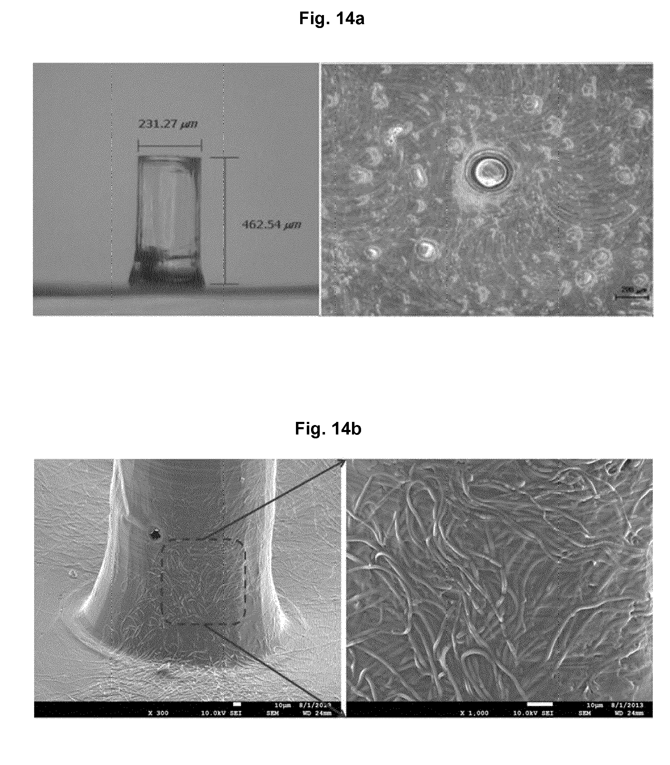

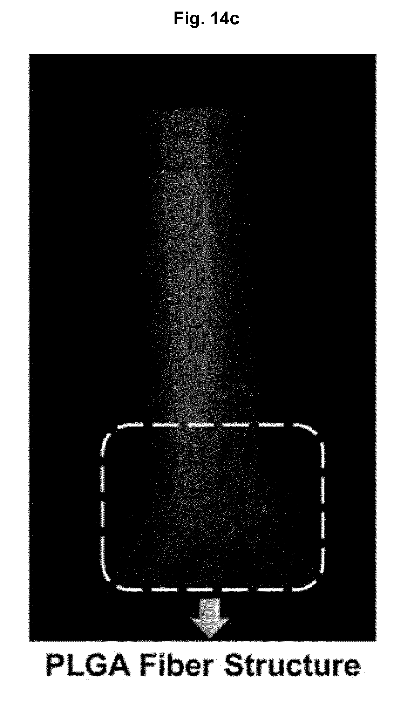

Hydrophobic fibers are favorable for sustained release due to the prolonged biodegradability of the fibers. However, these fibers are not able to homogeneously mix with the hydrophilic polymers which are used for dissolving microneedle fabrication such as sodium carboxymethyl cellulose (CMC), polyvinyl pyrrolidone (PVP), hyaluronic acid (HA), polyvinyl alcohol (PVA), hydroxypropylmethyl cellulose (HPMC) and so on. Therefore, the core hydrophobic fiber was coated with a hydrophilic polymer (outer shell) so that this composite fiber microstructure can form a homogenous fibrous gel with any of the hydrophilic polymers mentioned above. Thus, the fibrous gel was molded by centrifugation or casting method to form high strength microstructure for sustained cutaneous drug delivery. Figure 14 shows that the fibrous micropass array was fabricated by centrifugation molding method with the PDMS mold (Hole size: height 500 µm, diameter 300 µm). Optical microscope image of fibrous micropass shows the PLGA fiber structure inside fibrous CMC micropass base layer. Morever, it also can be confirmed by the top view of SEM image of fibrous CMC micropass and base layer (Figure 14).

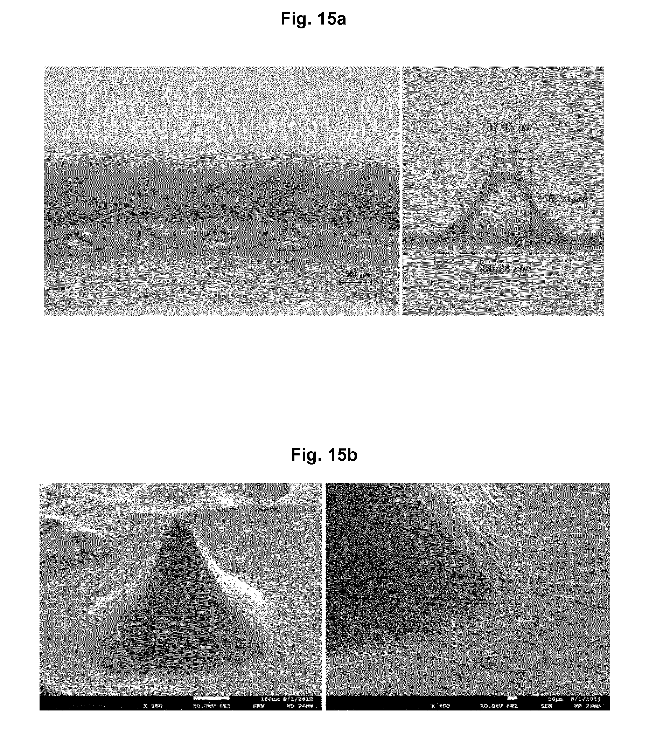

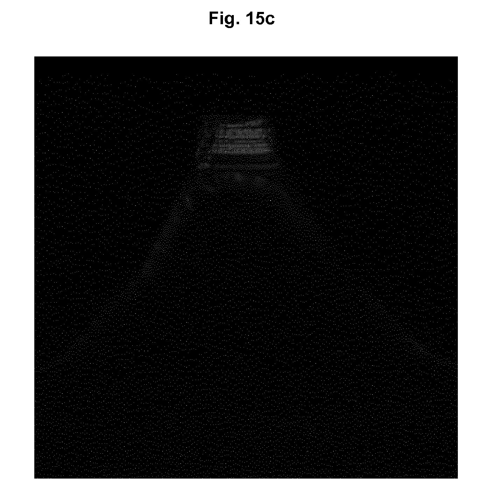

Figure 15 shows that the fibrous micropass array was fabricated by centrifugation molding method with the PDMS mold (Hole size: height 400 µm, diameter 500 µm). Optical microscope image of fibrous micropass shows the PLGA fiber structure inside fibrous CMC micropass base layer. Morever, it also can be confirmed by the top view of SEM image of fibrous CMC micropass and base layer (Figure 15).

Having described a specific embodiment of the present invention, it is to be understood that variants and modifications thereof falling within the spirit of the invention may become apparent to those skilled in this art, and the scope of this invention is to be determined by appended claims and their equivalents.

## Claims

1. A method for fabricating a three-dimensional porous fibrous microstructure, comprising:
(a) injecting a polymer solution into an injecting member including a syringe pump and a spinneret;
(b) spinning the polymer solution, which is injected into the injecting member, through the spinneret using the syringe pump together with the application of voltage, thereby obtaining a polymer fiber;
(c) collecting the polymer fiber into a mold; and
(d) drying the polymer fiber, and then separating the dried polymer fiber from the mold, thereby obtaining a three-dimensional porous fibrous microstructure.

2. The method according to claim 1, wherein the polymer solution corresponds to (i) a single polymer solution injected into a single injecting member; (ii) two or more polymer solutions which are respectively injected into two or more injecting members and individually spun through spinnerets of the respective injecting members; or (iii) two or more polymer solutions which are respectively injected into two or more injecting members and spun through a coaxial spinneret in which a spinneret of one injecting member is inserted into a spinneret of the other injecting member.

3. The method according to claim 2, wherein the polymer solution corresponds to (i) a single polymer solution injected into a single injecting member; (ii) a hydrophilic polymer solution and a hydrophobic polymer solution which are respectively injected into two or more injecting members and individually spun through spinnerets of the respective injecting members; or (iii) a hydrophilic polymer solution and a hydrophobic polymer solution which are respectively injected into two or more injecting members and spun through a coaxial spinneret in which a spinneret of one injecting member is inserted into a spinneret of the other injecting member.

4. The method according to claim 3, wherein the hydrophilic polymer solution is selected from the group consisting of sodium carboxymethyl cellulose (CMC), polyvinyl pyrrolidone (PVP), hyaluronic acid (HA), polyvinyl alcohol (PVA), and hydroxypropylmethyl cellulose (HPMC).

5. The method according to claim 3, wherein the hydrophobic polymer solution is poly(lactic-co-glycolic acid) (PVA).

6. The method according to claim 1, wherein the voltage is 5-20 kV.

7. The method according to claim 1, wherein the step (c) is performed by applying a downward pressure, a centrifugal force or a negative pressure to the mold.

8. The method according to claim 7, wherein the negative pressure is applied by an air suction system which is connected with a space inside the mold.

9. The method according to claim 1, wherein the step (c) is performed by contacting the mold with a fiber sheet loading the polymer fiber while applying a downward pressure to the mold.

10. The method according to claim 1, wherein the mold has a cylinder shape, a cone shape or a hemisphere shape.

11. A method for fabricating a three-dimensional porous fibrous microstructure, comprising:
(a) injecting a hydrophilic polymer solution and a hydrophobic polymer solution into two injecting members including syringe pumps and spinnerets;
(b) individually spinning the hydrophilic polymer solution and the hydrophobic polymer solution, which are injected into the respective injecting members, through the spinnerets using the syringe pumps together with the application of voltage, thereby obtaining a hydrophilic polymer fiber and a hydrophobic polymer fiber;
(c) collecting the hydrophilic polymer solution and the hydrophobic polymer into a mold;
(d) drying the polymer fibers, and then separating the dried polymer fibers from the mold, thereby obtaining a hybrid fibrous microstructure; and
(e) etching the hybrid fibrous structure with a water-soluble solvent, thereby obtaining a three-dimensional porous microstructure.

12. A method for fabricating a three-dimensional porous fibrous microstructure, comprising:
(a) injecting a polymer solution into an injecting member including a syringe pump and a spinneret;
(b) spinning the polymer solution, which is injected into the injecting member, through the spinneret using the syringe pump together with the application of voltage, thereby obtaining a polymer fiber;
(c) collecting the polymer fiber into a mold;
(d) drying the polymer fiber, and then separating the dried polymer fiber from the mold, thereby obtaining a three-dimensional porous fibrous microstructure; and
(e) contacting the obtained three-dimensional porous fibrous microstructure with a high-strength polymer solution to allow the high-strength polymer solution to be loaded on the three-dimensional porous fibrous microstructure, thereby obtaining a three-dimensional porous fibrous microstructure having an enhanced strength.

13. A three-dimensional porous fibrous microstructure fabricated by the method according to any of claims 1, 11 and 12.

14. An apparatus for detecting a biological marker comprising the three-dimensional porous fibrous microstructure according to claims 13.

15. The apparatus according to claim 13, wherein a sensor for the biological marker is connected to the porous fibrous microstructure.

16. A drug delivery system comprising the three-dimensional porous fibrous microstructure according to claim 13.
